(19) Europäisches Patentamt
European Patent Office
Office européen des brevets

(11) Numéro de publication: **0 255 431 B1**

# (12) FASCICULE DE BREVET EUROPEEN

(45) Date de publication de fascicule du brevet: **23.10.91**

(51) Int. Cl.[5]: **C12N 9/12**, C12P 21/08, A61K 39/395, G01N 33/577

(21) Numéro de dépôt: **87401729.6**

(22) Date de dépôt: **24.07.87**

## (54) Thymidine kinase de type foetal purifiée.

(30) Priorité: **31.07.86 FR 8611101**

(43) Date de publication de la demande: **03.02.88 Bulletin 88/05**

(45) Mention de la délivrance du brevet: **23.10.91 Bulletin 91/43**

(84) Etats contractants désignés: **AT BE CH DE ES FR GB GR IT LI LU NL SE**

(56) Documents cités:
**FR-A- 2 096 206**

**CHEMICAL ABSTRACTS, vol. 101, no. 17, 22 octobre 1984, page 535, résumé no. 149485y, Columbus, Ohio, US; L.M. BANKS et al.: "Monoclonal antibodies to herpes simplex virus thymidine kinase", & J. GEN. VIROL. 1984, 65(9), 1625-30**

(73) Titulaire: **LABORATOIRES DEBAT Société anonyme dite:
60 rue de Monceau
F-75008 Paris(FR)**

(72) Inventeur: **Jouan, Pierre Nicolas
Le Chêne Morand
F-35510 Cesson-Sévigné(FR)**

(74) Mandataire: **Clisci, Serge et al
S.A. FEDIT-LORIOT CONSEILS EN PROPRIETE INDUSTRIELLE 38, avenue Hoche
F-75008 Paris(FR)**

EP 0 255 431 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne en tant que nouveau produit industriel la thymidine kinase de type foetal purifiée. Elle concerne également le mode d'obtention de ce produit et plus particulièrement son procédé de purification. Ce produit est utile (i) dans le domaine des dosages, notamment celui des dosages immunologiques mettant en oeuvre des réactions antigène-anticorps, et (ii) en thérapeutique dans l'obtention d'anticorps intervenant en tant que médicaments.

ART ANTERIEUR

On sait que la thymidine kinase (en abrégé TK), enzyme responsable de la phosphorylation de la thymidine en thymidine-5'-monophosphate (en abrégé d.TMP), joue un rôle important dans la synthèse de l'ADN et, par conséquent, dans la multiplication cellulaire. Son activité est élevée dans les tissus en voie de prolifération ou de régénération et dans les cellules infectées par des virus. On sait également que deux isoenzymes de la TK ont été mises en évidence dans les cellules eucaryotes : la thymidine kinase de type foetal (désignée ci-après "thymidine kinase foetale" ou TK-F) et la thymidine kinase de type adulte (désignée ci-après "thymidine kinase adulte" ou TK-A).

La TK-F est présente dans les tissus embryonnaires et a été décelée dans les tissus cancéreux dans les cas de cancers hormonaux-dépendants, notamment les cancers du sein et de la prostate ; voir à ce sujet l'article de J.L. Javré et al., Bull. Cancer (Paris) 73 (No. 1), pages 8-16 (1986) intitulé "Mise en évidence de la thymidine kinase de type foetal dans les cancers du sein", et l'article de J.L. Javré et al. soumis à publication dans Cancer Research et intitulé "Presence of fetal thymidine kinase in human breast cancers".

On sait également que la synthèse de la TK-F in vivo est fortement augmentée par les hormones sexuelles dans leurs propres organes-cibles, la prostate et l'utérus ; voir à ce sujet l'article de M. Bourtourault et al., J. Steroid Biochem., 21 (No. 5), pages 613-620, (1984) intitulé : "Stimulation by 17$\beta$-estradiol of thymidine kinase activity in the rat uterus", et l'article de G. Gayet et al., The Prostate 7, pages 261-270, (1985) intitulé : "Induction of thymidine kinase of fetal type by androgens in the rat prostate".

On sépare la TK-F de la TK-A par électophorèse ou chromatographie au moyen d'une résine échangeuse d'anions,par exemple par électrophorèse sur gel d'acrylamide et, mieux, par chromatographie sur gel DEAE-cellulose ou gel DEAE-Sephadex commercialisé par la Société dite Pharmacia, l'isoenzyme foetale ayant une mobilité nulle ou faible et l'isoenzyme adulte une mobilité rapide.

La TK-F ainsi isolée, qui a une activité enzymatique par mg de l'ordre de 2 unités internationales, ne convient pas pour (i) réaliser des dosages plus précis, et surtout (ii) préparer des anticorps polyclonaux et mieux monoclonaux anti TK-F utiles dans le domaine du diagnostic, d'une part, et dans le domaine thérapeutique, d'autre part.

OBJET DE L'INVENTION

Suivant un premier aspect de l'invention on propose, en tant que produit industriel nouveau, une TK-F purifiée ayant une activité enzymatique nettement supérieure à la TK-F isolée jusqu'à présent selon les modalités de l'art antérieur.

Il existe un besoin en TK-F purifiée en particulier dans le domaine du diagnostic. En effet l'activité de la TK-F varie d'un cancer à l'autre. L'activité de la TK-F étant faible dans les cellules en phase Go et élevée dans les cellules en phase S, il y a tout lieu de penser que les cancers présentant une faible activité TK-F sont dans un état quiescent, alors que les cancers ayant une activité TK-F élevée sont en voie de prolifération. Le dosage de la TK-F dans les cancers du sein et de la prostate présente donc un grand intérêt pour mesurer l'état prolifératif des cancers. De plus le dosage de la TK-F au moyen d'anticorps anti TK-F levés contre la TK-F purifiée constitue un élément d'appréciation sur l'évolution de la maladie cancéreuse et par suite d'aide à l'orientation thérapeutique.

Suivant un second aspect de l'invention on préconise un procédé pour l'obtention de ladite TK-F purifiée, ledit procédé mettant en oeuvre le couplage de la TK-F, que l'on veut purifier, avec la thymidine, puis le clivage du complexe résultant (thymidine-TK-F) pour recueillir la TK-F purifiée.

Suivant encore un autre aspect de l'invention on préconise l'utilisation de la TK-F purifiée pour l'obtention d'anticorps anti TK-F, ces anticorps étant destinés à un usage diagnostic, d'une part, et à un usage thérapeutique, d'autre part. On a en effet trouvé de façon surprenante que l'administration d'anticorps anti TK-F chez des patients atteints d'un cancer hormono-dépendant (notamment un cancer du sein ou de la prostate) a un effet thérapeutique bénéfique.

## DESCRIPTION DETAILLEE DE L'INVENTION

Conformément à l'invention on préconise, en tant que produit industriel nouveau, une TK-F purifiée, qui n'avait pas encore été obtenue jusqu'à présent, utile dans le domaine des dosages et celui de l'obtention d'anticorps anti TK-F, ladite TK-F purifiée étant caractérisée en ce qu'elle a une activité enzymatique spécifique par mg supérieure ou égale à 5000 unités, de préférence supérieure ou égale à 8000 unités, et mieux supérieure ou égale à 10 000 unités.

Le Rf électrophorétique (à pH d'environ 8,6), en milieu non dénaturant (par exemple dans les tampons A et B décrits ci-après),est d'environ 0,15 pour une TK-F purifiée ayant une activité enzymatique spécifique par mg de l'ordre d'environ 11.800 unités.

L'activité enzymatique spécifique est exprimée, comme cela est l'habitude en unités enzymatiques par mg de protéine. Une unité de TK-F dite internationale correspond à 1 nanomole de nucléotides synthétisés par mg de protéine et par minute. En d'autres termes l'activité enzymatique peut être exprimée en nmoles/mg de protéine/min.

Pour comparaison on verra ci-après à l'exemple 1 que

- l'activité en milieu biologique d'origine est de 1,03 nmoles/mg protéine/min. ;
- l'activité après chromatographie sur DEA-Sephadex est de 2,08 nmoles/mg protéine/min. ;
- l'acitivité de la TK-F purifiée est de 11 875 nmoles/mg protéine/min.

Le procédé de préparation de la TK-F purifiée, que l'on préconise selon l'invention, dans lequel on réalise le couplage de la TK-F, que l'on veut purifier, avec la thymidine, puis le clivage du complexe thymidine-TK-F résultant, et caractérisé en ce que

a) on réalise le couplage thymidine-TK-F, par mise en contact de la TK-F à purifier avec de la thymidine préalablement fixée sur un premier support convenable,

b) on réalise l'élution de la TK-F ainsi liée au moyen d'un éluant convenable contenant de la thymidine, puis

c) on effectue le clivage du complexe thymidine-TK-F ainsi élué au moyen d'un second support qui est différent du premier support et adsorbe sélectivement la thymidine dudit complexe thymidine-TK-F.

Le meilleur mode de mise en oeuvre de ce procédé consiste en une chromatographie d'affinité qui comprend un mécanisme dit de compétition. Ainsi au stade a) on fait passer la TK-F que l'on veut purifier sur une résine appropriée préalablement couplée avec de la thymidine. La thymidine liée à ladite résine fixe la TK-F qui est retenue, alors que les impuretés de nature protéinique qui polluent ou souillent la TK-F ne se fixant pas à la thymidine ne sont pas retenues.

Au stade b) la TK-F fixée au support par l'intermédiaire de la thymidine préalablement couplée audit support, est éluée au moyen d'un éluant contenant de la thymidine libre. Le stade b) comprend le mécanisme dit de compétition, la TK-F liée à la thymidine fixée au support se couplant avec la thymidine libre de l'éluant. L'éluat ainsi recueilli contient le complexe thymidine-TK-F ainsi formé.

Au stade c) on destabilise ledit complexe thymidine-TK-F en adsorbant la thymidine sur un second support sélectif de la thymidine.

En pratique le support préféré du stade a) est un gel d'Epoxy-Sepharose commercialisé par la société dite Pharmacia sous la nomenclature d' Epoxy-Sepharose 6B. Ce gel est préalablement couplé à la thymidine selon une méthode connue, notamment celle décrite par P. Gröbner et al. J. Biol. Chem., 259, pages 8012-8014, (1984) ou une méthode similaire. Le support préféré du stade c) pour fixer la thymidine est une composition comprenant du charbon, du dextran et de la gélatine. La TK-F purifiée est ensuite isolée notamment par centrifugation de l'éluat qui la renferme avec ledit second support utilisé au stade c).

La TK-F de départ est une substance protéinique d'origine animale (obtention à partir de tissu foetal animal, notamment de foie de foetus de rongeur tel que le rat), d'origine humaine (obtention à partir de tissu cancéreux humain), ou encore d'origine synthétique (obtention par génie génétique notamment).

La TK-F ainsi purifiée est utile selon l'invention pour l'obtention d'anticorps anti TK-F, de préférence des anticorps monoclonaux plutôt que des anticorps polyclonaux. Ces anticorps sont obtenus selon une méthode classique connue en soi. Par exemple on innocule ladite TK-F purifiée à une souris, prélève la rate de l'animal immunisé, extrait les lymphocytes de la rate, fusionne lesdits lymphocytes avec des cellules de myélome de souris en croissance exponentielle, à raison de 5 à 20, de préférence 10 lymphocytes par cellule de myélome, sélectionne les hybridomes secréteurs ainsi obtenus afin d'obtenir des anticorps monoclonaux réagissant sélectivement avec la TK-F mais pas avec la TK-A, développe des tumeurs ascitiques chez la souris avec des clones sélectionnés, récupère les liquides d'ascite contenant les anticorps, purifie les anticorps monoclonaux ainsi obtenus.

Pour réaliser des dosages soit afin de détecter la présence ou l'absence de TK-F dans un tissu, soit encore d'évaluer quantitativement la teneur en TK-F dans ledit tissu on fixe l'anticorps anti TK-F sur un

support approprié (billes de verre, de polystyrène, parois de récipient, etc.) pour obtenir un matériel

(1) Support-anti TK-F

on met ensuite ce matériel en contact avec un milieu susceptible de contenir de la TK-F, la présence de ladite TK-F donnant le complexe

(2) Support-anti TK-F-TK-F

Ce complexe constitue un "signal" que l'on "amplifie" selon une méthode connue en soi pour visualisation et/ou détection avec un conjugué marqué anti

**TK-F***

[par exemple, méthode EIA (notamment EIA-sandwich ou EIA-compétition) selon laquelle on couple le complexe 2 avec un conjugué enzymatique approprié, méthode RIA comprenant le couplage dudit complexe 2 avec un conjugué marqué au moyen d'un isotope radioactif, méthode de fluorescence comprenant le couplage dudit complexe 2 avec un conjugué marqué à la fluorescéine]. L'amplification peut également être réalisée par agglutination ou encore selon une méthode ERICA mettant en oeuvre des cellules.

Selon l'invention on préconise des trousses ou kits de dosage comprenant les moyens réactifs requis pour mettre en oeuvre des réactions antigène-anticorps, notamment les réactions suivantes :

(a) Support—anti TK-F + TK-F ⟶ support—anti TK-F—TK-F

(b) Support—anti TK-F—TK-F + anti TK-F* ⟶ support—anti TK-F—TK-F—anti TK-F*

Selon l'invention on préconise une composition thérapeutique renfermant en association avec un excipient physiologiquement acceptable, une quantité thérapeutiquement efficace d'anti TK-F.

On vise en particulier l'utilisation d'anticorps anti TK-F pour l'obtention d'un médicament anticancéreux destiné à une utilisation thérapeutique vis-à-vis des cancers du sein et de la prostate.

D'autre avantages et caractéristiques de l'invention seront mieux compris à la lecture qui va suivre d'exemples de réalisation , l'ensemble de ces éléments n'étant pas limitatifs mais donné à titre d'illustration.

EXEMPLE 1

Isolement et purification de TK-F de foie de foetus de rat

1) Homogénéisation

Les tissus de foie de foetus de rat sont homogénéisés au Polytron dans la solution tampon : Tris-10 mM, $MgCl_2$ 10 mM, NaF 5 µM et β-mercaptoéthanol 0,4 mM à pH 7,5 (Tampon A).

2) Préparation du cytosol

L'homogénat résultant est centrifugé successivement à 800 x g et 12 000 x g pendant 10 min. dans une centrifugeuse réfrigérée. Le surnageant obtenu à 12 00 x g est, ensuite, centrifugé à 105 000 x g pendant 45 min. à +5°C dans une ultra-centrifugeuse Beckman, type L8-55. Le surnageant obtenu à 105 000 x g (cytosol) constitue la source de TK-F. Un dosage de l'activité enzymatique spécifique de la TK-F est effectué sur une partie aliquote du cytosol; cette activité est de 1,03 mmoles/mg/min.

3) Isolement de la TK-F

La TK-F est isolée par chromatographie sur gel de DEAE-Sephadex A50 (commercialisé par la société dite Pharmacia). Le gel de Sephadex est mis à gongler dans le tampon A. Après élimination des particules fines par sédimentation le gel est déposé dans une mini-colonne de 7 cm x 1 cm. 800 µl de gel sont introduits dans la colonne. Après préparation de la colonne de gel, une partie aliquote du cytosol est déposée sur le gel. La TK-F est éluée par passages successifs de 4 volumes (4 x 500 µl) de tampon A. Les élutions successives sont effectuées par centrifugation de la colonne à 200 x g dans une centrifugeuse réfrigérée. L'élution-centrifugation accélère l'élution et évite la dénaturation de la TK-F. Un dosage de l'activité enzymatique de la TK-F est effectué sur une partie aliquote de l'éluat et l'éluat restant est lyophilisé. A ce stade l'activité enzymatique est de 2,08 nmoles/mg/min.

4) Purification de la TK-F

La purification est effectuée par chromatographie d'affinité sur un gel d'époxy-Sépharose 6B (commercialisé par la société dite Pharmacia) couplé à la thymidine. Le couplage de la thymidine au gel de Sépharose est effectué en milieu alcalin, à pH 12,0 et à 25° C pendant 24 h selon une technique dérivée de celle de P. Gröbner et al. précité. Le taux de couplage de la thymidine est de 9 µM par ml de gel. Le gel est lavé par du tampon Tris 10 mM, $MgCl_2$ 10 mM, NaF 5 µm, β-mercaptoéthanol 0,4 mM, ATP 1 mM, à pH 7,5 (Tampon B). 3 ml de gel sont déposés dans une colonne de 10,6 cm de hauteur et 0,6 cm de diamètre. Le lyophilisat renfermant la TK-F est repris par de l'eau distillée et déposé sur le gel d'affinité. La pénétration de l'échantillon est effectuée à la vitesse de 1,2 ml par heure.

Le gel d'affinité est lavé par du tampon B pour éliminer les protéines non fixées sur le gel. Leur élution est effectuée à la vitesse de 1,2 ml par heure, puis de 4 ml par heure. La totalité des protéines non retenues par le gel sont éluées par un volume de 24 ml du tampon B.

L'élution de la TK-F est effectuée par passage, sur le gel d'affinité de 4,5 ml de tampon : Tris 10 mM, $MgCl_2$ 10 mM, NaF 5 µM, β-mercaptoéthanol 0,4 mM, ATP 1 mM et thymidine radio-inerte 10 mM,à pH 7,5. L'élution est effectuée avec un débit de 0,5 ml par heure. L'éluat ainsi obtenu contient le complexe thymidine-TK-F.

5) Elimination de la thymidine radio-inerte

L'élimination de la thymidine radio-inerte est effectuée par passage de l'éluat obtenu précédemment sur un culot de charbon-dextranne-gélatine (charbon 1% en poids, dextran T70 0,1% en poids et gélatine 0,2% en poids dans du tampon Tris-HCl 10 mM à pH 7,5). Le mélange est agité au vortex pendant 10 min. à 0° C puis centrifugé à 800 x g pendant 10 min. Le surnageant renferme la TK-F purifiée. Son dosage est effectué sur une partie aliquote du surnageant. L'activité enzymatique de la TK-F ainsi purifiée est de 11 875 nmoles/mg/min.

Le rendement en activité total est de 7,4 %. Le taux de purification est de l'ordre de 11 530 fois, et le Rf électrophorétique de la TK-F purifiée (déterminé en milieu non dénaturant) est de 0,15à pH 8,6.

EXEMPLE 2
Technique de dosage de l'activité de la TK-F

A 20 µl de l'extrait renfermant la TK-F, on ajoute 5 µl de tampon Tris-HCl (pH 7,8) 200 mM renfermant 76 mM de $MgCl_2$, 160 mM de phosphoglycérate, 40 mM d'ATP et 40 à 50µM de [2-$^{14}$C]-thymidine (activité spécifique 54-58 mCi/mmole ; i.e. environ 2 x $10^8$ à 2,15 x $10^8$ Bq/mmole). Le milieu réactionnel est incubé à 37° C pendant 25 min puis la réaction est stoppée par addition de 10 µl de $ClO_4H$ 2,1 M et centrifugation à 2400 x g pendant 10 min. 10 µl du surnageant de centrifugation sont, ensuite, utilisés pour la séparation, par électrophorèse haute tension, des nucléotides synthétisés. La séparation des d-TMP,d-TDP et d-TTP (thymidine-monophosphate, -diphosphate, et -triphosphate) est effectuée sur papier Whatman 3MM, en tampon citrate à pH 4,1 sous 47 volts par cm de longueur de papier, pendant 30 min et à 3° C. Après séchage de l'électrophorégramme puis repérage sous U.V. des nucléotides témoins, le papier est découpé en bandelettes de 2,5 cm de largeur par 0,5 cm de hauteur et celles-ci sont transférées dans des fioles de comptage de la radioactivité renfermant 10 ml de liquide scintillant (Ready-solv EP, Beckman).

EXEMPLE 3
Contrôle de la pureté de la TK-F par électrophorèse sur gel de polyacrylamide

L'électrophorèse est effectuée dans un gel de polyacrylamide en gradient linéaire de 10 à 15 % en

présence de dodécylsulfate de sodium (SDS), sur plaque verticale. La migration des protéines est effectuée dans du tampon Tris 12 g‰, glycine 28,8 g‰, et SDS 0,1 g % à pH 8,7 sous 70 volts pendant 12 h.

Les protéines provenant du cytosol et de l'éluat DEAE-Sephadex sont colorées par une solution de Bleu de Coomassie R 250 à la concentration de 0,5 g dans 250 ml de méthanol à 90 %. La coloration des protéines obtenues après chromatographie d'affinité est effectuée par la méthode de coloration à l'argent selon la technique de P. Tunon and K.E. Johansson, J. Biol. Chem. and Biophys. Methods., 9, pages 171-179, (1984).

EXEMPLES 4 et 5

En procédant comme indiqué à l'exemple 1, mais en remplaçant les tissus de foie de foetus de rat par des tissus cancéreux humains de sein et de prostate on obtient une TK-F purifiée analogue à celle du foie de foetus de rat, et ayant sensiblement les mêmes propriétés (activité enzymatique) Rf, que ladite TK-F purifiée de foie de foetus de rat.

On observe en particulier pour l'ensemble des produits des exemples 1, 4 et 5 que contrairement à celle de la TK-A, l'activité de la TK-F n'est pas inhibée par le d-CTP, et que la mobilité électrophorétique de la TK-F en gel de polyacrylamide et à pH 8,6 est nulle ou très faible alors que la TK-A présente une mobilité rapide. Ces deux isoenzymes ont, aussi des comportements différents sur colonne de DEAE-Sephadex ou DEAE-cellulose. La TK-F n'est pas retenue sur ce type de résine et elle est éluée par des solutions de faible force ionique. La TK-A est retenue sur ces résines et ne peut être éluée que par des solutions de force ionique élevée. Le comportement électrophorétique et chromatographique indique donc que la TK-F a un caractère alcalin et la TK-A un caractère acide. Le pH de la TK-F serait, dans l'état actuel des connaissances, de 9,0-9,7 et celui de la TK-A de 5,0-5,7.

Suivant l'invention, on préconise en tant que produits industriels nouveaux les anticorps anti TK-F levés contre la TK-F purifiée conforme à l'invention obtenue à partir d'une source quelconque, notamment une TK-F purifiée d'origine animale ou humaine.

Comme indiqué ci-dessus , les anticorps anti TK-F, notamment les anticorps monoclonaux anti TK-F, levés contre la TK-F purifiée sont particulièrement utiles dans le domaine des dosages. De préférence, la TK-F purifiée utilisée pour l'obtention desdits anticorps monoclonaux est d'origine animale (notamment le tissu de foie foetal de rongeur) ou humaine (notamment de tissu cancéreux humain).

**Revendications**

**Revendications pour les Etats contractants suivants : BE, CH, DE, FR, GB, IT, LI, LU, NL, SE**

**1.** Thymidine kinase de type foetal (TK-F) purifiée, caractérisée en ce qu'elle a une activité enzymatique par mg supérieure ou égale à 5000 unités, de préférence supérieure ou égale à 8000 unités, et mieux supérieure ou égale à 10 000 unités.

**2.** TK-F purifiée selon la revendication 1, caractérisée en ce que son activité enzymatique par mg est de l'ordre d'environ 11 800 unités.

**3.** TK-F purifiée selon la revendication 2, caractérisée en ce que son Rf électrophorétique en milieu non dénaturant, est de l'ordre de 0,15 à pH d'environ 8,6.

**4.** Procédé de préparation de TK-F purifiée selon l'une quelconque des revendications 1 à 3; dans lequel on réalise le couplage de la TK-F, que l'on veut purifier, avec la thymidine, puis le clivage du complexe thymidine-TK-F résultant, caractérisé en ce que

a) on réalise le couplage thymidine-TK-F, par mise en contact de la TK-F à purifier avec de la thymidine préalablement fixée sur un premier support convenable,

b) on réalise l'élution de la TK-F ainsi liée au moyen d'un éluant convenable contenant de la thymidine, puis

c) on effectue le clivage du complexe thymidine-TK-F ainsi élué au moyen d'un second support qui est différent du premier support et adsorbe sélectivement la thymidine dudit complexe thymidine-TK-F.

**5.** Procédé selon la revendication 4, caractérisé en ce que le premier support est l'époxy-sépharose.

**6.** Procédé selon la revendication 4, caractérisé en ce que l'élution des impuretés protéiniques non

retenues sur le premier support couplé à la thymidine est effectuée avec un tampon Tris 10 mM, $MgCl_2$ 10 mM, NaF 5 $\mu$M, $\beta$-mercaptoéthanol 0,4 mM et ATP 1 mM à pH 7,5.

7. Procédé selon la revendication 4, caractérisé en ce que l'élution de la TK-F fixée à la thymidine du support couplé à ladite thymidine est effectuée avec un tampon Tris 10 mM, $MgCl_2$ 10 mM, NaF 5 $\mu$M, $\beta$-mercaptoéthanol 0,4 mM, ATP 1 mM et thymidine 10 mM à pH 7,5.

8. Procédé selon la revendication 4, caractérisé en ce que le second support est une composition comprenant 1 % en poids de charbon, 0,1 % en poids de dextran et 0,2 % en poids de gélatine dans du tampon Tris-HCl 10 mM à pH 7,5.

9. Utilisation de la TK-F purifiée selon l'une quelconque des revendications 1 à 3, caractérisée en ce que ladite TK-F est utilisée pour l'obtention d'anticorps, de préférence monoclonaux, anti TK-F.

10. Utilisation d'anticorps monoclonaux anti TK-F, selon la revendication 9 pour l'obtention d'un médicament anticancéreux destiné à une utilisation en thérapeutique vis à vis des cancers de la prostate et du sein.

11. Anticorps anti TK-F, caractérisé en ce qu'il est levé contre la TK-F purifiée selon la revendication 1.

12. Anticorps anti TK-F selon la revendication 11, utile dans le domaine des dosages, caractérisé en ce qu'il est monoclonal, et, en ce qu'il a été levé contre la TK-F purifiée selon la revendication 1 obtenue à partir d'une source animale ou humaine.

**Revendications pour les Etats contractants suivants : AT, ES, GR**

1. Thymidine kinase de type foetal (TK-F) purifiée, caractérisée en ce qu'elle a une activité enzymatique par mg supérieure ou égale à 5000 unités, de préférence supérieure ou égale à 8000 unités, et mieux supérieure ou égale à 10 000 unités.

2. TK-F purifiée selon la revendication 1, caractérisée en ce que son activité enzymatique par mg est de l'ordre d'environ 11 800 unités.

3. TK-F purifiée selon la revendication 2, caractérisée en ce que son Rf électrophorétique, en milieu non dénaturant, est de l'ordre de 0,15 à pH d'environ 8,6.

4. Procédé de préparation de TK-F purifiée selon l'une quelconque des revendications 1 à 3; dans lequel on réalise le couplage de la TK-F, que l'on veut purifier, avec la thymidine, puis le clivage du complexe thymidine-TK-F résultant, caractérisé en ce que
   a) on réalise le couplage thymidine-TK-F, par mise en contact de la TK-F à purifier avec de la thymidine préalablement fixée sur un premier support convenable,
   b) on réalise l'élution de la TK-F ainsi liée au moyen d'un éluant convenable contenant de la thymidine, puis
   c) on effectue le clivage du complexe thymidine-TK-F ainsi élué au moyen d'un second support qui est différent du premier support et adsorbe sélectivement la thymidine dudit complexe thymidine-TK-F.

5. Procédé selon la revendication 4, caractérisé en ce que le premier support est l'époxy-sépharose.

6. Procédé selon la revendication 4, caractérisé en ce que l'élution des impuretés protéiniques non retenues sur le premier support couple à la thymidine est effectuée avec un tampon Tris 10 mM. $MgCl_2$ 10 mM, NaF 5 $\mu$M, $\beta$-mercaptoéthanol 0,4 mM et ATP 1 mM, à pH 7,5.

7. Procédé selon la revendication 4, caractérisé en ce que l'élution de la TK-F fixée à la thymidine du support couplé à ladite thymidine est effectuée avec un tampon Tris 10 mM, $MgCl_2$ 10 mM. NaF 5 $\mu$M, $\beta$-mercaptoéthanol 0,4 mM, ATP 1 mM et thymidine 10 mM à pH 7,5.

8. Procédé selon la revendication 4, caractérisé en ce que le second support est une composition

comprenant 1 % en poids de charbon, 0.1 % en poids de dextran et 0.2 % en poids de gélatine dans du tampon Tris-HCl 10 mM à pH 7,5.

9. Utilisation de la TK-F purifiée selon l'une quelconque des revendications 1 à 3, caractérisée en ce que ladite TK-F est utilisée pour l'obtention d'anticorps, de préférence monoclonaux, anti TK-F.

10. Anticorps anti TK-F, caractérisé en ce qu'il est levé contre la TK-F purifiée selon la revendication 1.

11. Anticorps anti TK-F selon la revendication 10, utile dans le donaine des dosages, caractérisé en ce qu'il est monoclonal, et, en ce qu'il a été levé contre la TK-F purifiée selon la revendication 1 obtenue à partir d'une source animale ou humaine.

**Claims**

**Claims for the following Contracting States : BE, CH, DE, FR, GB, IT, LI, LU, NL, SE**

1. A purified thymidine kinase of fetal type (TK-F) having an enzymatic activity per mg greater than or equal to 5000 units, preferably greater than or equal to 8000 units and particularly preferably greater than or equal to 10,000 units.

2. The purified TK-F according to claim 1, whose enzymatic activity per mg is of the order of about 11,800 units.

3. The purified TK-F according to claim 2, whose electrophoretic Rf in a non-denaturing medium is of the order of 0.15 at a pH of about 8.6.

4. A method for the preparation of purified TK-F according to any one of claims 1 to 3, wherein the TK-F to be purified is coupled with thymidine and the resulting thymidine/TK-F complex is then split, which comprises:
   a) coupling the TK-F with thymidine by bringing the TK-F to be purified into contact with thymidine which has been fixed beforehand to a first suitable support,
   b) eluting the TK-F, bound in this way, by means of a suitable eluent containing thymidine, and then
   c) splitting the thymidine/TK-F complex, eluted in this way, by means of a second support which is different from the first support and selectively adsorbs the thymidine from the said thymidine/TK-F complex.

5. The method according to claim 4, wherein the first support is epoxy-Sepharose.

6. The method according to claim 4, wherein the protein-type impurities not retained on the first support coupled with thymidine are eluted with the following buffer: 10 mM Tris, 10 mM $MgCl_2$, 5 $\mu$M NaF, 0.4 mM $\beta$-mercaptoethanol and 1 mM ATP at pH 7.5.

7. The method according to claim 4, wherein the TK-F fixed to the thymidine of the support coupled with the said thymidine is eluted with the following buffer: 10 mM Tris, 10 mM $MgCl_2$, 5 $\mu$M NaF, 0.4 mM $\beta$-mercaptoethanol, 1 mM ATP and 10 mM thymidine at pH 7.5.

8. The method according to claim 4, wherein the second support is a composition comprising 1% by weight of charcoal, 0.1% by weight of dextran and 0.2% by weight of gelatin in 10 mM Tris-HCl buffer at pH 7.5.

9. Use of the purified TK-F according to any one of claims 1 to 3, wherein the said TK-F is used for the preparation of anti-TK-F antibodies, the said antibodies preferably being monoclonal.

10. Use of monoclonal anti-TK-F antibodies according to claim 9, for the preparation of an anticancer drug for use in therapy in the treatment of prostate and breast cancers.

11. An anti-TK-F antibody which is raised against the purified TK-F according to claim 1.

12. The anti-TK-F antibody according to claim 11, useful in the field of quantitative analyses, which is

monoclonal and which has been raised against the purified TK-F according to claim 1, obtained from an animal or human source.

**Claims for the following Contracting States : AT, ES, GR**

1. A purified thymidine kinase of fetal type (TK-F) having an enzymatic activity per mg greater than or equal to 5000 units, preferably greater than or equal to 8000 units and particularly preferably greater than or equal to 10,000 units.

2. The purified TK-F according to claim 1, whose enzymatic activity per mg is of the order of about 11,800 units.

3. The purified TK-F according to claim 2, whose electrophoretic Rf in a non-denaturing medium is of the order of 0.15 at a pH of about 8.6.

4. A method for the preparation of purified TK-F according to any one of claims 1 to 3, wherein the TK-F to be purified is coupled with thymidine and the resulting thymidine/TK-F complex is then split, which comprises:
   a) coupling the TK-F with thymidine by bringing the TK-F to be purified into contact with thymidine which has been fixed beforehand to a first suitable support,
   b) eluting the TK-F, bound in this way, by means of a suitable eluent containing thymidine, and then
   c) splitting the thymidine/TK-F complex, eluted in this way, by means of a second support which is different from the first support and selectively adsorbs the thymidine from the said thymidine/TK-F complex.

5. The method according to claim 4, wherein the first support is epoxy-Sepharose.

6. The method according to claim 4, wherein the protein-type impurities not retained on the first support coupled with thymidine are eluted with the following buffer: 10 mM Tris, 10 mM $MgCl_2$, 5 µM NaF, 0.4 mM $\beta$-mercaptoethanol and 1 mM ATP at pH 7.5.

7. The method according to claim 4, wherein the TK-F fixed to the thymidine of the support coupled with the said thymidine is eluted with the following buffer: 10 mM Tris, 10 mM $MgCl_2$, 5 µM NaF, 0.4 mM $\beta$-mercaptoethanol, 1 mM ATP and 10 mM thymidine at pH 7.5.

8. The method according to claim 4, wherein the second support is a composition comprising 1% by weight of charcoal, 0.1% by weight of dextran and 0.2% by weight of gelatin in 10 mM Tris-HCl buffer at pH 7.5.

9. Use of the purified TK-F according to any one of claims 1 to 3, wherein the said TK-F is used for the preparation of anti-TK-F antibodies, the said antibodies preferably being monoclonal.

10. An anti-TK-F antibody which is raised against the purified TK-F according to claim 1.

11. The anti-TK-F antibody according to claim 10, useful in the field of quantitative analyses, which is monoclonal and which has been raised against the purified TK-F according to claim 1, obtained from an animal or human source.

**Patentansprüche**
**Patentansprüche für folgende Vertragsstaaten : BE, CH, DE, FR, GB, IT, LI, LU, NL, SE**

1. Gereinigte fötale Thymidinkinase (TK-F),
   dadurch **gekennzeichnet,**
   daß sie eine enzymatische Aktivität pro mg oberhalb oder gleich 5000 Einheiten, vorzugsweise oberhalb oder gleich 8000 und insbesondere oberhalb oder gleich 10000 Einheiten aufweist.

2. Gereinigte TK-F gemäß Anspruch 1,
   dadurch **gekennzeichnet,**

daß sie pro mg eine enzymatische Aktivität in der Größenordnung von etwa 11800 Einheiten aufweist.

**3.** Gereinigte TK-F gemäß Anspruch 2,
dadurch **gekennzeichnet,**
daß sie einen elektrophoretischen Rf-Wert, in einem nicht denaturierenden Milieu, in der Größenordnung von 0,15 bei einem pH-Wert von etwa 8,6 aufweist.

**4.** Verfahren zur Herstellung von gereinigter TK-F gemäß einem der Ansprüche 1 bis 3, wobei man die zu reinigende TK-F mit Thymidin verbindet und den resultierenden Thymidin-TK-F-Komplex wieder spaltet,
dadurch **gekennzeichnet,**
daß man
a) die Verbindung Thymidin-TK-F durch Inberührungbringen der zu reinigenden TK-F mit dem Thymidin vornimmt, das vorher auf einem ersten Träger fixiert ist,
b) die Elution der so gebundenen TK-F mittels eines geeigneten thymidinhaltigen Extraktionsmittels durchführt und dann
c) die Trennung des so eluierten Thymidin-TK-F-Komplexes mittels eines zweiten Trägers vornimmt, der verschieden vom ersten Träger ist und der das Thymidin des Thymidin-TK-F-Komplexes selektiv adsorbiert.

**5.** Verfahren gemäß Anspruch 4,
dadurch **gekennzeichnet,**
daß der erste Träger Epoxy-Sepharose ist.

**6.** Verfahren gemäß Anspruch 4,
dadurch **gekennzeichnet,**
daß die Elution der proteinischen Verunreinigungen, die nicht vom ersten, mit dem Thymidin verbundenen Träger festgehalten werden, durchgeführt wird mittels eines Puffers aus Tris 10 mM, $MgCl_2$ 10 mM, NaF 5 µM, β-Mercaptoethanol 0,4 mM und ATP 1 mM bei einem pH-Wert von 7,5.

**7.** Verfahren gemäß Anspruch 4,
dadurch **gekennzeichnet,**
daß die Elution der TK-F, gebunden an das Thymidin im mit dem Thymidin verbundenen Träger, durchgeführt wird, mit einem Puffer aus Tris 10 mM, $MgCl_2$ 10mM, NaF 5 µM β-Mercaptoethanol 0,4 mM, ATP 1 mM und Thymidin 10mM bei einem ph-Wert von 7,5.

**8.** Verfahren gemäß Anspruch 4,
dadurch **gekennzeichnet,**
daß der zweite Träger eine Zusammensetzung ist mit einem Gehalt an 1 Gew.-% Kohle, 0,1 Gew.-% Dextran und 0,2 Gew.-% Gelatine in einem Puffer aus Tris-HCl 10mM bei einem pH-Wert von 7,5.

**9.** Verwendung der gereinigten TK-F gemäß einem der Ansprüche 1 bis 3,
dadurch **gekennzeichnet,**
daß die TK-F verwendet wird zur Herstellung von Antikörpern, besonders monoklonalem, Anti-TK-F.

**10.** Verwendung des monoklonalen Anti-TK-F Antikörpers gemäß Anspruch 9, für die Herstellung von einem anticancerogenen Medikament, bestimmt für den therapeutischen Einsatz gegen Protata- und Brust-Krebs.

**11.** Anti-TK-F Antikörper,
dadurch **gekennzeichnet,**
daß er eingesetzt worden ist gegen die gereinigte TK-F gemäß Anspruch 1.

**12.** Anti-TK-F Antikörper gemäß Anspruch 11, geeignet für den Dosierungsbereich,
dadurch **gekennzeichnet,**
daß er monoklonal ist und eingesetzt war gegen das gereinigte TK-F gemäß Anspruch 1, erhalten aus einer tierischen oder menschlichen Quelle.

**Patentansprüche für folgende Vertragsstaaten : AT, ES, GR**

**1.** Gereinigte fötale Thymidinkinase (TK-F), dadurch **gekennzeichnet,** daß sie eine enzymatische Aktivität pro mg oberhalb oder gleich 5000 Einheiten, vorzugsweise oberhalb oder gleich 8000 und insbescndere oberhalb oder gleich 10000 Einheiten aufweist.

**2.** Gereinigte TK-F gemäß Anspruch 1, dadurch **gekennzeichnet,** daß sie pro mg eine enzymatische Aktivität in der Größenordnung von etwa 11800 Einheiten aufweist.

**3.** Gereinigte TK-F gemäß Anspruch 2, dadurch **gekennzeichnet,** daß sie einen elektrophoretischen Rf-Wert, in einem nicht denaturierenden Milieu, in der Größenordnung von 0,15 bei einem pH-Wert von etwa 8,6 aufweist.

**4.** Verfahren zur Herstellung von gereinigter TK-F gemäß einem der Ansprüche 1 bis 3, wobei man die zu reinigende TK-F mit Thymidin verbindet und den resultierenden Thymidin-TK-F-Komplex wieder spaltet, dadurch **gekennzeichnet,** daß man

a) die Verbindung Thymidin-TK-F durch Inberührungbringen der zu reinigenden TK-F mit dem Thymidin vornimmt, das vorher auf einem ersten Träger fixiert ist,

b) die Elution der so gebundenen TK-F mittels eines geeigneten thymidinhaltigen Extraktionsmittels durchführt und dann

c) die Trennung des so eluierten Thymidin-TK-F-Komplexes mittels eines zweiten Trägers vornimmt, der verschieden vom ersten Träger ist und der das Thymidin des Thymidin-TK-F-Komplexes selektiv adsorbiert.

**5.** Verfahren gemäß Anspruch 4, dadurch **gekennzeichnet,** daß der erste Träger Epoxy-Sepharose ist.

**6.** Verfahren gemäß Anspruch 4, dadurch **gekennzeichnet,** daß die Elution der proteinischen Verunreinigungen, die nicht vom ersten, mit dem Thymidin verbundenen Träger festgehalten werden, durchgeführt wird mittels eines Puffers aus Tris 10 mM, $MgCl_2$ 10 mM NaF 5µM $\beta$-Mercaptoethanol 0,4 mM und ATP 1 mM bei einem pH-Wert von 7,5.

**7.** Verfahren gemäß Anspruch 4, dadurch **gekennzeichnet,** daß die Elution der TK-F, gebunden an das Thymidin im mit dem Thymidin verbundenen Träger, durchgeführt wird, mit einem Puffer aus Tris 10 mM $MgCl_2$ 10mM NaF 5 µM, $\beta$-Mercaptoethanol 0,4 mM ATP 1 mM und Thymidin 10mM bei einem ph-Wert von 7,5.

**8.** Verfahren gemäß Anspruch 4, dadurch **gekennzeichnet,** daß der zweite Träger eine Zusammensetzung ist mit einem Gehalt an 1 Gew.-% Kohle, 0,1 Gew.-% Dextran und 0,2 Gew.-% Gelatine in einem Puffer aus Tris-HCl 10mM bei einem pH-Wert von 7,5.

**9.** Verwendung der gereinigten TK-F gemäß einem der Ansprüche 1 bis 3, dadurch **gekennzeichnet,** daß die TK-F verwendet wird zur Herstellung von Antikörpern, besonders monklonalem, Anti-TK-F.

**10.** Anti-TK-F Antikörper, dadurch **gekennzeichnet,** daß er eingesetzt worden ist gegen die gereinigte TK-F gemäß Anspruch 1.

11. Anti-TK-F Antikörper gemäß Anspruch 10 geeignet für den Dosierungsbereich, dadurch **gekennzeichnet,** daß er monoklonal ist und eingesetzt war gegen das gereinigte TK-F gemäß Anspruch 1 erhalten aus einer tierischen oder menschlichen Quelle.